# EUROPÄISCHE PATENTSCHRIFT

(11) **EP 4 138 960 B1**
(45) Veröffentlichungstag und Bekanntmachung des Hinweises auf die Patenterteilung: **15.04.2026**
(21) Anmeldenummer: 21720274.6
(22) Anmeldetag: 22.04.2021
(51) Int. Cl.: A61M 5/31, A61M 5/34, A61J 1/06

(54) **SPRITZE UND SPRITZENKÖRPER**
SYRINGE AND SYRINGE BODY
SERINGUE ET CORPS DE SERINGUE

(30) Priorität: 24.04.2020 EP 20171394
(43) Veröffentlichungstag der Anmeldung: 01.03.2023
(73) Patentinhaber: Fresenius Kabi Deutschland GmbH, 61352 Bad Homburg (DE)
(72) Erfinder: BRANDENBURGER, Torsten, 61440 Oberursel (DE); SCHÖNHOFEN, Michael, 61440 Oberursel (DE)
(74) Vertreter: Fresenius Kabi Deutschland GmbH
(86) Internationale Anmeldenummer: PCT/EP2021/060468
(87) Internationale Veröffentlichungsnummer: WO 2021/214192

(56) Entgegenhaltungen:
- EP-A1- 3 342 441
- US-A1- 2011 046 603
- US-B1- 6 656 164

## Beschreibung

### Gebiet der Erfindung

Die Erfindung betrifft eine mit einer medizinischen Flüssigkeit vorbefüllte Spritze umfassend einen Spritzenkörper mit Luer-Lock-Anschluss sowie ein Verfahren zur Herstellung des Spritzenkörpers.

### Hintergrund der Erfindung

Vorbefüllte Spritzen, die auch als Einwegspritzen bezeichnet werden, können einen Spritzenkörper aus Kunststoff umfassen. Zur Abgabe der enthaltenen medizinischen Flüssigkeit besitzt der Spritzenkörper an seiner Stirnseite eine Düse. Die Düse dient z.B. zum Anschluss einer Nadel oder eines Schlauchs eines Überleitsystems. Verbreitet sind insbesondere Spritzen mit männlichem Luer-Lock-Anschluss. Dabei ist die genormt konisch ausgebildete Düse von einer Hülse mit einem Innengewinde umgeben.

Insbesondere bei vorbefüllten Spritzen sollte ein geeignetes Kunststoffmaterial verschiedene Anforderungen erfüllen.

Das Kunststoffmaterial sollte für möglichst viele verschiedene medizinische Flüssigkeiten geeignet sein. Es soll insbesondere eine Diffusion von Bestandteilen der medizinischen Flüssigkeit in das Material und auch ein Auslaugen von Materialkomponenten durch die medizinische Flüssigkeit weitgehend, auch bei langer Lagerung, vermieden werden. Zudem sollte ein geeignetes Kunststoffmaterial auch autoklavierbar sein.

Als besonders geeignete Materialien haben sich Cycloolefin-Copolymere etabliert. Diese sind zum einen gut im Spritzgussverfahren zu verarbeiten. Zum anderen zeichnen sie sich durch eine hohe Steifigkeit und hohe Härte bei gleichzeitig relativ niedriger Dichte aus. Weiter sind derartige Materialien amorph und hochtransparent. Weiterhin besitzen sie eine geringe Wasseraufnahme sowie eine geringe Wasserdampfdurchlässigkeit.

Diese Kunststoffe können allerdings verhältnismäßig spröde sein. Cycloolefin-Copolymere haben beispielsweise verglichen mit Polypropylen oder Polyethylen eine verhältnismäßig niedrige Bruchdehnung. Dies kann zum Beispiel dazu führen, dass bei unsachgemäß starkem Festziehen des Anschlusses eines Überleitsystems das Luer-Lock-Gewinde der Spritze beschädigt wird. Aufgrund der Sprödigkeit des Materials kann sich das Material nicht nennenswert plastisch verformen, so dass eine derartige Beschädigung recht abrupt erfolgen kann. So hat der Anwender fast keine taktile Rückmeldung, dass er den Anschluss zu stark festzieht.

Um dieses Problem zu reduzieren, sieht die Offenlegungsschrift DE 10 2017 112 823 A1 eine Verstärkung der Gewindehülse durch Rippen vor. Hierdurch wird die mechanische Stabilität des Gewindeanschlusses verbessert.

Die Offenlegungsschrift EP 3 342 441 A1 schlägt vor, den Gewindeanschluss, gegenüber dem Spritzenkörper, aus einem anderen Material bereitzustellen. Es ist insbesondere vorgesehen, dass das Anschlussstück aus weicherem Material an der der Vorderseite des Spritzengrundkörper angespritzt wird.

### Aufgabe der Erfindung

Der Erfindung liegt demgegenüber die Aufgabe zugrunde, die genannten Probleme des Standes der Technik zumindest zu reduzieren.

Es ist insbesondere eine Aufgabe der Erfindung, eine mit einer medizinischen Flüssigkeit vorbefüllte Spritze aus Kunststoff bereitzustellen, welche eine Innenwand aus einem Material mit hoher chemischer Beständigkeit und guter Barrierewirkung umfasst, und welche gleichzeitig mit einem sehr stabilen Gewindeanschluss aus Kunststoff versehen ist.

### Zusammenfassung der Erfindung

Die Aufgabe der Erfindung wird durch einen Spritzenkörper nach Anspruch 1 sowie eine Spritze mit diesem Spritzenkörper gelöst.

Bevorzugte Ausführungsformen und Weiterbildungen der Erfindung sind dem Gegenstand der abhängigen Ansprüche, der Beschreibung sowie den Zeichnungen zu entnehmen.

Die Erfindung einen Spritzenkörper mit einer Düse und einem Gewindeanschluss,
wobei der Spritzenkörper ein Innenteil umfasst, das eine Innenwand des Spritzenkörpers ausbildet und das ein Cycloolefin, insbesondere ein Cyclooloefin-Copolymer oder ein Cycloolefinpolymer, als erstes Material umfasst, und wobei der Gewindeanschluss als Teil eines Außenteils aus einem Kunststoff ausgebildet ist, welches als zweites Material ein anderes Material als das Cycloolefin umfasst, wobei sich das Außenteil zumindest abschnittsweise um die Seitenwand des Spitzenkörpers erstreckt.

Die Erfindung basiert also auf der Grundidee, dass der Spritzenkörper aus einem Innenteil und einem Außenteil gebildet wird, wobei Innenteil und Außenteil aus unterschiedlichen Materialien ausgebildet sind.

Die Erfindung sieht insbesondere vor, dass zumindest die Innenwand, vorzugsweise die gesamte Innenwand des Spritzenkörpers aus einem Cycloolefin gebildet wird.

Unter Cycloolefin im Sinne der Erfindung werden sämtliche Cycloolefin-Copolymere verstanden, welche durch katalysierte Copolymerisation von Cycloolefinen gewonnen werden.

Im Sinne der Erfindung werden unter Cycloolefinen auch Materialien verstanden, welche durch eine ringöffnende Salzmetathese gewonnen werden. Diese werden genau genommen nicht als Cycloolefin-Copolymer, sondern als Cycloolefinpolymer bezeichnet.

Das erste Material besteht vorzugsweise zu mindestens 50 Gewichts%, besonders bevorzugt zu mindestens 90 Gewichts% aus einem Cycloolefin.

Derartige Materialien sind amorph, transparent und gehen in wesentlich geringerem Maße als andere Kunststoffe Wechselwirkungen mit der eingefüllten medizinischen Flüssigkeit, insbesondere mit einer wässrigen medizinischen Flüssigkeit, ein.

Insbesondere kann es sich um sog. Chrystal-Clear-Polymere handeln.

Das Außenteil, welches den Gewindeanschluss umfasst, kann dagegen aus einem anderen Kunststoff bestehen, insbesondere einem Kunststoff, der mechanisch stabiler ist.

Dadurch dass sich das Außenteil um die Seitenwand des Spritzenkörpers erstreckt, werden beim Einschrauben eines korrespondierenden Anschlusses keine Drehmomente über den Gewindeanschluss an das Innenteil übertragen.

Das Außenteil dient als Griff, um die Spitze beim Einschrauben eines Konnektors in den Gewindeanschluss festzuhalten.

Der Benutzer hält also die Spritze insbesondere an der Seitenwand am Außenteil fest, welches gleichzeitig den Gewindeanschluss umfasst. So werden keine Drehmomente auf das Innenteil übertragen.

Innenteil und Außenteil neigen daher weniger dazu, sich zu lösen.

Das Innenteil kann mit dem Außenteil umspritzt sein.

Auch eine formschlüssige Verbindung, z.B. durch Verrasten ist gemäß einer anderen Ausführungsform vorgesehen.

Innenteil und Außenteil können auch miteinander verschweißt oder verklebt sein.

Bei einer Ausführungsform sind Innenteil und Außenteil ineinandergesteckt. Es genügen bereits Reibungskräfte zwischen den Wänden, um Innenteil und Außenteil zusammenzuhalten.

Vorzugsweise erstreckt sich das Außenteil um die gesamte Seitenwand des Innenteils.

Das Außenteil kann Aussparungen aufweisen, insbesondere wenn dieses opak ausgebildet ist.

Vorzugsweise ist das Außenteil aber transparent.

Bei einer Ausführungsform der Erfindung ist ein proximaler Griff der Spritze bzw. des Spritzenkörpers Teil des Außenteils. Insbesondere umfasst nur das Außenteil einen proximalen, radial nach außen ragenden Griff.

So kann auf einfache Weise eine Spritze bereitgestellt werden, bei welcher auch der Griff aus dem mechanisch robusteren Material ausgebildet ist.

Die Wandstärke des Innenteils beträgt bei einer Ausführungsform der Erfindung das 0,3 bis 3fache, vorzugsweise das 0,5 bis 1,5fache, der Wandstärke der angrenzenden Wand des Außenteils. Dies gilt insbesondere für die Seitenwand. Insbesondere haben Innenteil und Außenteil in etwa dieselbe Wandstärke.

Das Innenteil hat also eine relativ hohe Wandstärke und führt daher zu einer guten Barrierewirkung, insbesondere für Wasserdampf und/oder Sauerstoff.

Die Gesamtwandstärke, insbesondere im Bereich der Seitenwand kann z.B**.** zwischen 0,3 mm und 5 mm, vorzugsweise zwischen 0,6 mm und 2,5 mm liegen.

Bei einer Ausführungsform der Erfindung erstreckt sich das Außenteil auch um die Düse des Innenteils.

So schiebt sich beim Anschließen des Konnektors das weibliche Anschlussstück über das Außenteil. Axiale, auf das Innenteil wirkende Kräfte, die das Innenteil vom Außenteil lösen könnten, werden so vermieden.

Als Fertigspritze befüllt, greift ein Verschluss für die Düse, beispielsweise eine Dichtung vorzugsweise in die Düse. So kommt das Material des Außenteils bei Lagerung der Spritze nicht mit der Flüssigkeit im Innenvolumen der Spritze in Kontakt.

Bei einer Weiterbildung der Erfindung umfasst das Innenteil eine Strukturierung an der Vorderseite seiner Stirnwand, in die eine komplementäre Strukturierung an der Rückseite des Außenteils eingreift.

Der Spritzenkörper umfasst zumindest einen zweischichtigen Bereich, innerhalb dessen erstes und zweites Material eine ineinandergreifende Strukturierung umfassen.

Aufgrund des Innenteils und des Außenteils ist der Spritzenkörper zumindest bereichsweise zweischichtig aufgebaut.

Diese Ausführungsform der Erfindung ist sowohl für eine Spritze vorgesehen, bei der Innenteil und Außenteil formschlüssig miteinander verbunden sind, als auch für eine Spritze mit stoffschlüssig mit dem Innenteil verbundenem Außenteil.

Unter einer Strukturierung wird ein regelmäßiges oder unregelmäßiges Höhenprofil des Innenteils mit Erhebungen oder Vertiefungen verstanden. In die Vertiefungen greift das, bespielweise im Spritzgussverfahren angespritzte, zweite Material des Außenteilsein.

Die an einer Kontaktfläche oder Grenzfläche innerhalb des zweischichtigen Bereichs aneinander angrenzenden Flächen aus dem ersten und dem zweiten Material umfassen also jeweils ein Oberflächenrelief mit einer Profilstruktur, die durch das jeweils andere Material zumindest teilweise aufgefüllt ist

Es hat sich herausgestellt, dass dadurch die Neigung der zwei Schichten zu einer Trennung der Materialien, insbesondere beim Autoklavieren und/oder bei mechanischen Belastungen zu trennen, deutlich reduziert werden kann.

Spannungen, etwa aufgrund unterschiedlicher thermischer Ausdehnungskoeffizienten der Materialien, werden aufgrund der Berge und Täler nicht nur im Wesentlichen radial entlang einer glatten Fläche verteilt, sondern werden teilweise in axiale Richtung umgeleitet.

Der lineare Wärmeausdehnungskoeffizient bei Cycloolefin-Copolymeren (bei Standardbedingungen) liegt in der Regel bei unter 60 x 10⁻⁶ x K⁻¹. Polypropylene haben dagegen beispielsweise einen höheren thermischen Längenausdehnungskoeffizienten, insbesondere von über 150 x 10⁻⁶ x K⁻¹.

Trotz der Festigkeit und einer gewissen Sprödigkeit des Cycloolefin-Copolymers kommt es, insbesondere bei einem Autoklavierungsprozess durch unterschiedliche thermische Ausdehnung der beiden Materialien, nicht zu einem Aufbrechen der stoffschlüssigen Verbindung.

Weiter wird durch die Strukturierung die Größe der Oberfläche einer Grenzfläche zwischen dem ersten und dem zweiten Material vergrößert. Vorzugsweise ist diese Grenzfläche gegenüber einer unstrukturierten Ausgestaltung um mindestens 20 % größer.

Schließlich kann durch die Strukturierung ein Formschluss zwischen dem ersten und dem zweiten Material bereitgestellt werden, insbesondere gegenüber einem Drehmoment zwischen den Bauteilen, wenn ein Konnektor mit dem Gewindeanschluss verbunden wird.

Hierdurch kann auf sehr einfache Weise eine verbesserte mechanische Verbindung bereitgestellt werden.

Weiter wird durch die Strukturierung die Größe der Oberfläche einer Grenzfläche bzw. Kontaktfläche zwischen dem ersten und dem zweiten Material vergrößert. Vorzugsweise ist diese Grenz- oder Kontaktfläche gegenüber einer unstrukturierten Ausgestaltung um mindestens 20% größer.

Schließlich kann durch die Strukturierung ein Formschluss zwischen dem ersten und dem zweiten Material bereitgestellt werden, insbesondere gegenüber einem Drehmoment zwischen dem Innenteil und dem Außenteil.

Hierdurch kann auf sehr einfache Weise eine verbesserte mechanische Verbindung bereitgestellt werden.

Weiterhin ist die Herstellung einer derartigen Spritze nicht mit wesentlich erhöhtem Aufwand verbunden. So braucht lediglich für die Herstellung des Innenteils ein Spritzgusswerkzeug verwendet zu werden, welches ein Negativprofil in der Form der Profilierung des Innenteils umfasst.

Soweit im Folgenden von einer Strukturierung die Rede ist, kann es sich dabei sowohl um die Strukturierung des Innenteils als auch um die Strukturierung des Außenteils handeln, insbesondere an ihren Stirnwänden.

Diese Strukturierungen haben vorzugsweise eine komplementäre Ausgestaltung.

Die Erhebungen können als regelmäßige und/oder unregelmäßige Erhebungen ausgebildet sein. Auch die Vertiefungen können als regelmäßige und/oder unregelmäßige Vertiefungen ausgebildet sein.

Bei einer Ausführungsform der Erfindung sind die Erhebungen und/oder die Vertiefungen als konzentrische Ringe ausgebildet. Bei einer derartigen Profilierung verlaufen die Erhebungen und die Vertiefungen insbesondere radial von innen nach außen. Dadurch kommt es insbesondere überall zu einer gleichmäßigen Umlenkung von Spannungen in axialer Richtung.

Die Erhebungen oder Vertiefungen können insbesondere einen im Wesentlichen dreieckigen Querschnitt aufweisen. Bei dieser Ausführungsform ist die Profilierung als ein Sägezahnprofil ausgebildet. Aber auch andere Strukturen, wie beispielsweise eine Wellenform, insbesondere eine Sinusform, ineinandergreifende Noppen, etc. sind möglich.

Bei einer Ausführungsform der Erfindung sind Elemente der Strukturierung des ersten Materials konisch ausgebildet. Die Elemente der Strukturierung, z.B. die Stege oder Noppen, verjüngen sich nach oben. Hierdurch wird ein Bereitstellen der Strukturierung der zweiten Schicht durch ein Anspritzen des zweiten Materials erleichtert. Insbesondere wird die Neigung Blasenbildung im an die Strukturelemente angrenzenden Bereich reduziert.

Bei einer Ausführungsform der Erfindung umfasst die Strukturierung an der Vorderseite der Stirnwand des Innenteils zumindest einen Steg und/oder eine Nut, der bzw. die sich von der Düse, zumindest abschnittsweise, in Richtung der Seitenwand erstreckt. So wird gleichsam eine Verdrehsicherung bereitgestellt. Die Elemente der Strukturierung können auch als Noppen und/oder Keile ausgebildet sein.

In einer Ausführungsform kann die Strukturierung der Stirnwand des Innenteils zumindest einen, vorzugsweise eine Vielzahl, von sich radial erstreckenden Stegen und/oder Nuten aufweisen. Die Stege oder Nuten können hierbei einen beliebigen Querschnitt haben. Vorzugsweise sind sie jedoch mit einem Sägezahnprofil ausgebildet.

Aufgrund der radialen Ausrichtung bilden Steg und Nut jeweils einen Formschluss, der wie eine Verdrehsicherung wirkt.

Insbesondere kann die Strukturierung in der Draufsicht eine fächerartige und/oder sternartige Ausgestaltung aufweisen. Beispielsweisewerden die Stäbe eines Fächers durch sich radial erstreckende Stege gebildet und die Bespannung wird durch das sich ringförmig ersteckende Profil zwischen den Stegen gebildet. Der Fächer erstreckt sich um den Gewindeanschluss und nimmt dabei 360° der Stirnfläche ein.

Die Strukturierung kann zwischen 3 und 16, vorzugsweise zwischen 6 und 10 sich radial erstreckende und um den Umfang verteilte Stege und/oder Nuten aufweisen.

Der durch die Strukturierung bereitgestellte Formschluss dient insbesondere der Verbesserung der Festigkeit der Verbindung bei einer Drehmomentbelastung, wie beispielsweise bei einem starken Einschrauben des Anschlusses.

Bei einer Weiterbildung der Erfindung ist das Außenteil aus dem zweiten Material um den Gewindeanschluss herum verdickt gegenüber einem radial angrenzenden Bereich des Außenteils bzw. der Stirnwand des Außenteils aus dem zweiten Material ausgebildet.

Im Übergangsbereich vom Gewindeanschluss zur Stirnwand ist also das Material der zweiten Schicht durch eine Verdickung, wie beispielsweise eine Wulst oder eine Innenfase, verstärkt. Hierdurch wird die Gefahr des Abbrechens des Gewindeanschlusses bei zu starkem Festziehen reduziert.

Weiter wird insbesondere bei Verwendung eines weichen zweiten Materials die Verformung des Materials im Bereich der Stirnwand beim Festziehen reduziert, wodurch wiederum die Neigung eines Lösens der stoffschlüssigen Verbindung in diesem Bereich reduziert wird.

Das zweite Material ist vorzugsweise ein Kunststoff, insbesondere ein transparenter Kunststoff, welcher sich im Spritzgussverfahren verarbeiten lässt.

Vorzugsweise hat das zweite Material eine höhere Kerbschlagzähigkeit als das erste Material. Insbesondere hat das zweite Material eine mindestens 20%, vorzugsweise mindestens 50%, höhere Kerbschlagzähigkeit, als das erste Material.

Die Kerbschlagzähigkeit ist ein Materialkennwert, der die Neigung des Materials zu Rissbildung bei dynamischer Belastung definiert. Diese wird im Kerbschlagbiegeversuch bestimmt. Die dynamische Biegung durch die schlagartige Beanspruchung verursacht einen Bruch, oft ohne das bei langsamer Beanspruchung beobachtete Fließen des Werkstoffs.

Im Sinne der Erfindung werden alle Materialkennwerte, also insbesondere die Kerbschlagzähigkeit und die folgenden Materialkennwerte wie E-Modul und Härte, bei Standardbedingungen, also 20 °C und 50% Luftfeuchtigkeit, bestimmt.

Die Kerbschlagzähigkeit im Sinne der Erfindung wird nach DIN ISO 179-1 (11/2010) bestimmt.

Dabei wird ein von seiner Form genormter Prüfkörper mit einer Einkerbung durch ein Schlagpendel belastet. Das Schlagpendel kerbt mit einer definierten kinetischen Energie den Prüfkörper oder durchschlägt diesen. Im Anschluss wird die Kerbe gemessen oder bei Durchschlagung die Höhe festgehalten, zu der das Pendel zurückschwingt. Aus dem Gewicht des Schlagpendels und der Differenz der Pendelausgangs- und Endlage lässt sich die verbrauchte Schlagarbeit errechnen, wobei die Schlagarbeit das Produkt aus Probenquerschnitt und Kerbschlagzähigkeit darstellt.

Cycloolefine haben im Allgemeinen eine Kerbschlagzähigkeit von unter 3 kJ/m². Dahingegen sollte das zweite Material eine höhere Kerbschlagzähigkeit, insbesondere von über 3,5 kJ/m², bevorzugt von über 5 kJ/m², haben sollte.

Als zweites Material kann insbesondere ein Polypropylen verwendet werden.

Insbesondere teilkristallines Polypropylen hat gute mechanische Eigenschaften und ist gleichzeitig transparent.

Das zweite Material kann insbesondere weicher als das erste Material sein.

Insbesondere kann das zweite Material eine Härte Shore D (nach DIN ISO 7619-1 (2/2012) von unter 75, insbesondere von unter 70, haben.

Das Cycloolefin hat dagegen in einer Ausführungsform der Erfindung eine Härte Shore D von über 80.

Bei einer Ausführungsform der Erfindung hat das zweite Material ein kleineres Elastizitätsmodul als das erste Material.

Insbesondere beträgt das E-Modul des zweiten Materials (nach DIN ISO 527-1 (2/2019) zwischen 1000 und 1800 MPa. Das E-Modul des Cycloolefins kann insbesondere zwischen 1800 und 2200 MPa liegen.

Wichtige Kenngröße für das zweite Material ist insbesondere auch dessen Bruchdehnung.

Gemäß einer Ausführungsform der Erfindung hat das zweite Material eine mindestens 1,5fach, vorzugsweise eine mindestens 5fach und besonders bevorzugt eine mindestens 10fach höhere Bruchdehnung als das erste Material.

Die Bruchdehnung wird ebenfalls nach DIN ISO 527-1 bestimmt. Diese wird in % angegeben.

Das verwendete Cycloolefine kann z.B. eine Bruchdehnung von unter 5% haben, wohingegen beispielsweise ein verwendetes Polypropylen eine Bruchdehnung von 100% oder mehr haben kann.

Um den Gewindeanschluss zum Abbrechen zu bringen, muss das Material über die obere Streckgrenze hinaus verformt werden. Die anschließende Verformung im plastischen Bereich eines Materials mit hoher Bruchdehnung bemerkt ein geschulter Anwender in der Regel, so dass er erkennt, dass nunmehr die Belastbarkeitsgrenze des Anschlusses eigentlich schon überschritten ist.

In einer Ausführungsform der Erfindung ist die Strukturierung an der Vorderseite der Stirnwand des Innenteils insbesondere durch einen radial umlaufenden Steg begrenzt. Die Strukturierung endet innerhalb der Stirnwand des Innenteils.

Der Steg kann insbesondere durch einen vorderen Abschnitt der Seitenwand des Innenteils gebildet sein.

Gemäß einer Ausführungsform hat die Strukturierung des ersten und/oder des zweiten Materials eine maximale Strukturtiefe von über 0,1 mm, vorzugsweise von über 0,25 mm, und/oder von weniger als 1 mm, vorzugsweise weniger als 0,5 mm. Unter der maximalen Strukturtiefe wird der Höhenunterschied zwischen der Spitze einer Erhebung und dem Grund einer Vertiefung verstanden. Bei der Strukturierung handelt es sich also um eine Strukturierung der Größenordnung im Makrobereich, wodurch vorstehend beschriebene Kraftumlenkung effektiv erzielt wird.

Der Abstand Erhebung zu Erhebung bzw. Vertiefung zu Vertiefung, die sogenannte Strukturbreite, beträgt bei einer Ausführungsform der Erfindung mehr als 0,2 mm, vorzugsweise mehr als 0,5 mm, und/oder weniger als 3 mm, vorzugsweise weniger als 1,5 mm.

Weiter kann gemäß einer Ausführungsform der Erfindung das Verhältnis maximale Strukturtiefe zu Strukturbreite mehr als 0,1, vorzugsweise mehr als 0,25, betragen und/oder unter 1, vorzugsweise unter 0,5 liegen.

Die Wandstärke der Schicht aus dem ersten Material beträgt im Bereich der Stirnwand bei einer Ausführungsform der Erfindung das 0,3 bis 3fache, vorzugsweise das 0,5 bis 1,5fache, der Wandstärke der Schicht aus dem zweiten Material. Die Gesamtwandstärke, insbesondere im Bereich der zumindest abschnittsweise zweischichtig ausgebildeten Stirnwand, kann z.B. zwischen 0,3 mm und 5 mm, vorzugsweise zwischen 0,6 mm und 2,5 mm, liegen.

Im Bereich der Erfindung liegt auch eine Spritze umfassend einen Spritzenkörper gemäß einer der vorstehend beschriebenen Ausführungsformen, wobei die Spritze einen Stopfen aufweist, mit welchem die Düse verschlossen ist, einen Kolben und auch insbesondere eine Kolbenstage zum Abgeben der medizinischen Flüssigkeit über die Düse. Die Kolbenstange kann bereits an dem Kolben vormontiert sein oder separat bereitgestellt sein.

Die erfindungsgemäße Spritze ist vorzugsweise mit einer medizinischen Flüssigkeit befüllt, insbesondere mit einer solchen, die ein Medikament enthält.

Die Spritze ist in diesem Ausführungsbeispiel eine vorgefüllte Spritze.

Insbesondere befindet sich die Spritze in einer vorzugsweise sauerstoff-impermeablen Außenverpackung, beispielsweise einer aufreißbaren Folienverpackung. Die Spritze, vorzugsweise die verpackte Spritze, ist insbesondere autoklaviert.

Insbesondere befindet sich die Spritze in einer vorzugsweise sauerstoff-impermeablen Außenverpackung, beispielsweise einer aufreißbaren Folienverpackung.

Die Spritze wurde in dieser Außenverpackung autoklaviert, beispielsweise bei einer Temperatur von über 110°C, vorzugsweise über 120°C, und ist so vollständig steril.

Gemäß einer Ausführungsform ist die medizinische Flüssigkeit ein sauerstoffempfindliches Arzneistofffluid, beispielsweise eine Arzneistoffemulsion. Gemäß einer Ausführungsform ist oder umfasst das Arzneistofffluid Propofol, insbesondere eine Propofol-Emulsion. Propofol wird beschrieben durch den chemischen Namen 2,6-Diisopropylphenol (IUAPC).

Der Spritzenkörper kann beispielsweise durch ein Spritzgussverfahren hergestellt werden.

Dabei wird zunächst ein Innenteil aus einem Cycloolefin als erstes Material gespritzt.

Hierfür wird ein Spritzgusswerkzeug verwendet, welches das Negativ einer Strukturierung der Stirnwand des Innenteils umfasst, so dass ein Innenteil mit einer strukturierten Stirnwand entsteht.

Anschließend wird ein Außenteil mit einem Gewindeanschluss aus einem zweiten Material um das Innenteil gespritzt.

Im Bereich der strukturierten Stirnwand des Spritzenkörpers bildet das Außenteil hierbei automatisch eine komplementäre Strukturierung aus, welche, wie vorstehend ausgeführt, die mechanische Verbindung der beiden Materialien erheblich verbessert.

### Kurzbeschreibung der Zeichnungen

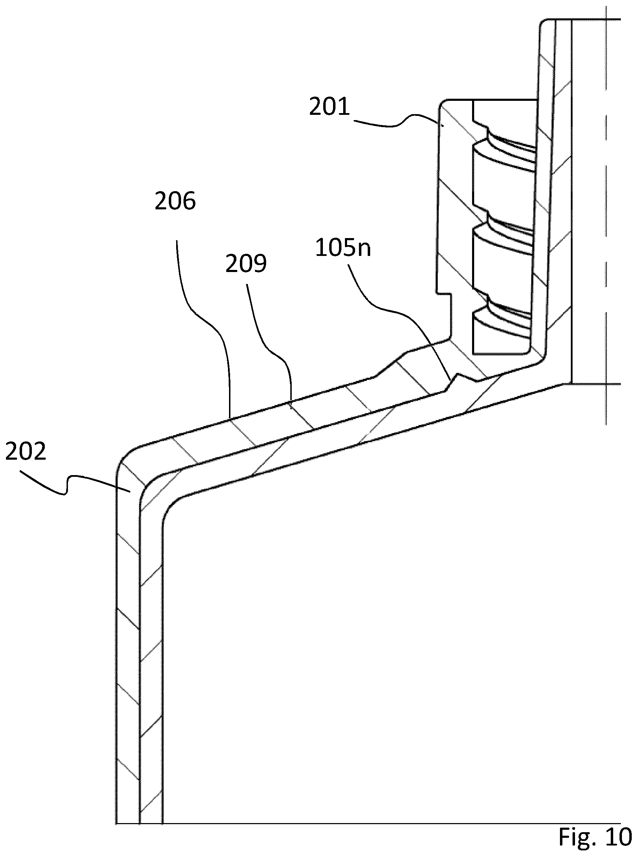

Der Gegenstand der Erfindung soll im Folgenden anhand von Ausführungsbeispielen gemäß Fig. 1 bis Fig. 10 näher erläutert werden.
Fig. 1 ist eine Seitenansicht eines Ausführungsbeispiels einer erfindungsgemäßen Spritze.
Fig. 2 ist eine axiale Schnittansicht entlang der Linie A-A der Fig. 1.
Fig. 3 ist eine axiale Schnittansicht des Spritzenkörpers.
Fig. 4 zeigt ein alternatives Ausführungsbeispiel eines Spritzenkörpers.
Fig. 5 ist eine Detailansicht des Bereichs D der Fig. 4
Fig. 6 ist eine perspektivische Ansicht des Kopfstücks der Spritze.
Fig. 7 ist eine perspektivische Ansicht des Kopfstücks des Innenteils.
Fig. 8 ist eine Draufsicht auf die Stirnwand des Spritzenkörpers.
Fig. 9 ist eine Schnittansicht entlang der Linie B gemäß Fig. 8.
Fig. 10 ist eine Schnittansicht entlang der Linie C gemäß Fig. 8.

### Detaillierte Beschreibung der Zeichnungen

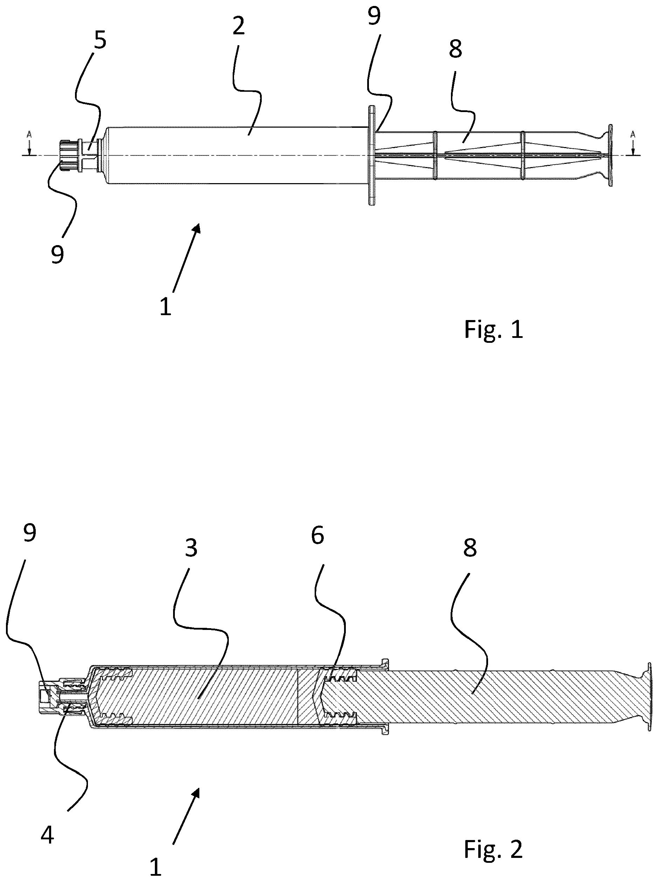

Fig. 1 zeigt in einer Seitenansicht ein Ausführungsbeispiel einer erfindungsgemäßen Spritze 1.

Die Spritze 1 umfasst einen Spritzenkörper 2 mit einem Gewindeanschluss 5 und einem proximalen Griff 9.

Wie in der axialen Schnittansicht gemäß Fig. 2 dargestellt, stellt der Spritzenkörper 2 ein Innenvolumen 3 bereit, dass mit einer medizinischen Flüssigkeit vorbefüllt ist. Ein Gasvolumen im Innenvolumen 3 gleicht Druckschwankungen aus und verhindert insbesondere, dass sich der Spritzenkörper 2 bei Druckschwankungen verformt.

Die Düse 4 ist mit einer Dichtung 9 verschlossen, die vorzugsweise in die Düse 4 hineingreift, so dass die Außenwand der Düse 4 zumindest in verschlossenem Zustand nicht mit der medizinischen Flüssigkeit in Kontakt kommt.

Düse 4 und Gewindeanschluss 5 bilden den Anschluss der Spritze 1, welcher insbesondere als männlicher Luer-Lock-Anschluss ausgebildet ist.

Die medizinische Flüssigkeit kann über die mit dem Kolben 6 verbundene Kolbenstange 8 durch die Düse 4 abgegeben werden.

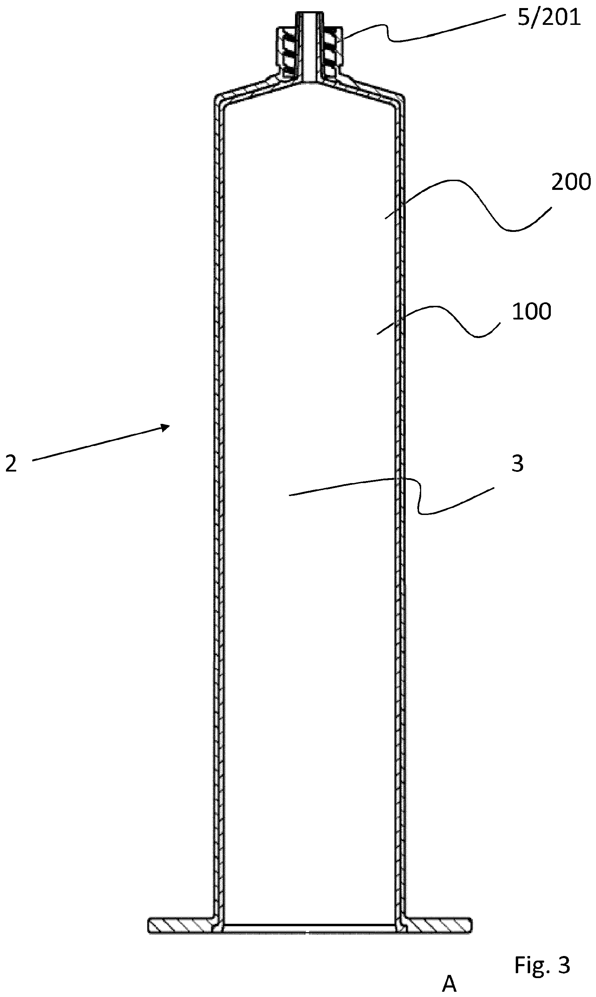

Fig. 3 zeigt in einer axialen Schnittansicht nur den Spritzenkörper 2.

Der Spritzenkörper 2 ist zweiteilig ausgebildet und umfasst ein Innenteil 100 aus Cycloolefin sowie ein Außenteil 200, welches gegenüber dem Innenteil 100 aus einem mechanisch stabileren Material ausgebildet ist, insbesondere aus einem Polypropylen.

Der Gewindeanschluss 5/201 wird allein durch das Außenteil 200 bereitgestellt.

Die Erfindung ist für Spritzen mit nahezu beliebigem Volumen, insbesondere von 1 ml bis 100 ml, vorgesehen.

In dem Ausführungsbeispiel gemäß Fig. 3 ist eine relativ große Spritze, insbesondere mit einem Innenvolumen 3 von etwa 50 ml, dargestellt.

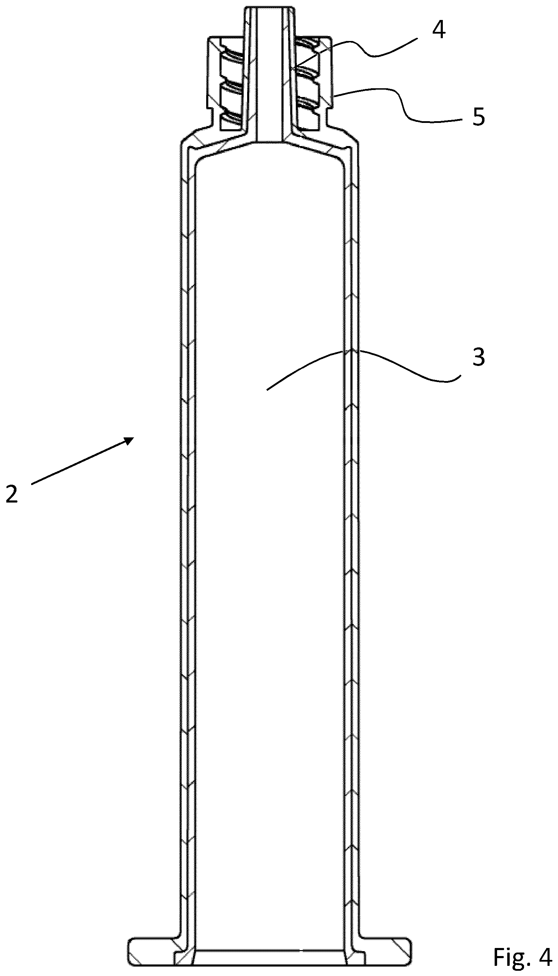

Fig. 4 ist eine axiale Schnittansicht einer alternativen Ausführungsform eines Spritzenkörpers 2.

Es handelt sich bei diesem Ausführungsbeispiel um eine relativ kleine Spritze, insbesondere mit einem Innenvolumen 3 von etwa 5 ml. Der grundsätzliche Aufbau des Spritzenkörpers 2 bleibt jedoch unverändert.

Dabei unterscheiden sich die Abmessungen des Anschlusses, bestehend aus Düse 4 und Gewindeanschluss 5, nicht.

Mithin hat der Gewindeanschluss 5 im Verhältnis zum Maximaldurchmesser des Spritzenkörpers 2 einen wesentlich größeren Durchmesser.

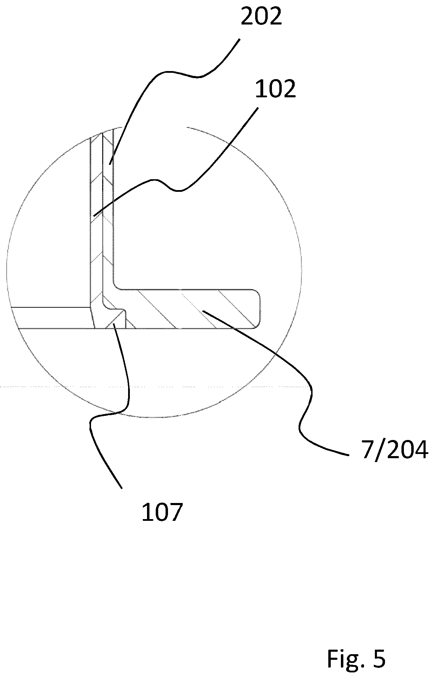

Fig. 5 ist eine Detaildarstellung des Bereichs D der Fig. 3, also des proximalen Endes des Spritzenkörpers 2.

Die Seitenwand des Spritzenkörpers 2 ist zweischichtig ausgebildet und wird aus der Seitenwand 102 des Innenteils und der angrenzenden Seitenwand 202 des Außenteils 200 gebildet.

Der Griff 7/204 besteht dagegen zumindest abschnittsweise nur aus dem Material des Außenteils 200.

In dieser Ausführungsform hat das Innenteil 100 einen proximalen Kragen 107, welcher in den Griff 204 hineinragt und z.B. die axiale Positionierung bei einer Ausführungsform, bei der Innenteil 100 und Außenteil 200 ineinandergeschoben werden, verbessert, oder der bei einer Ausführungsform, bei der das Innenteil 100 mit dem Außenteil 200 umspritzt wird, den Griff 6 steifer macht.

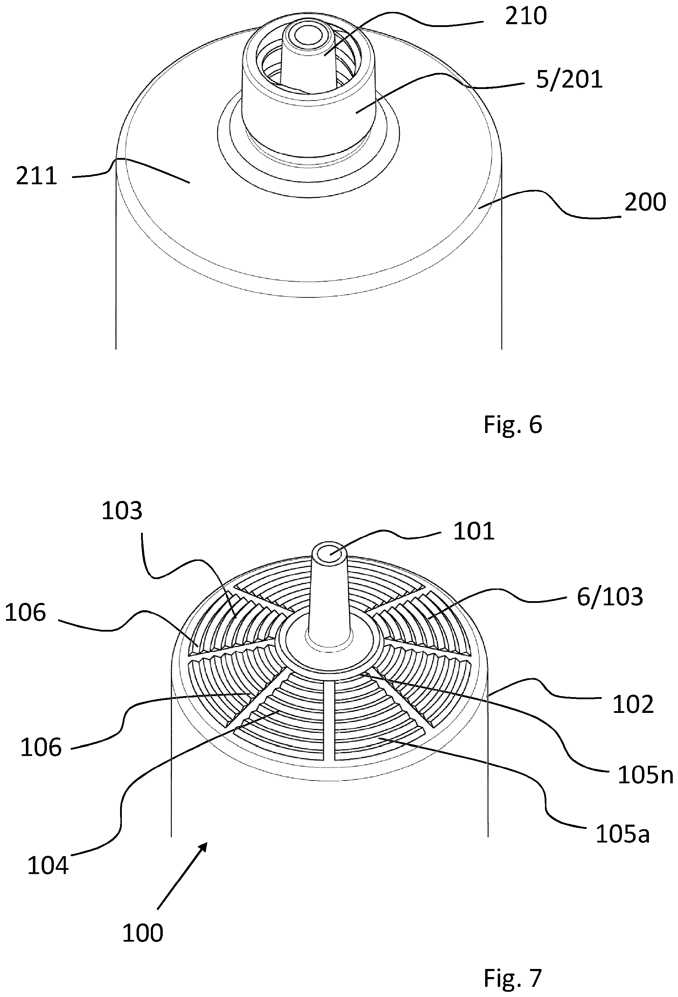

Fig. 6 zeigt in einer perspektivischen Ansicht das Kopfstück einer Spritze 1 gemäß eines Ausführungsbeispiels der Erfindung.

Der Spritzenkörper 2 wird, wie vorstehend ausgeführt, aus Innenteil 100 und Außenteil 200 gebildet, so dass in dieser Ansicht vom Innenteil 100 allenfalls die Innenwand der Düse 4 zu erkennen ist.

Der Gewindeanschluss 5/201 mit einem Innengewinde erstreckt sich um die Düse 4/210, welche zweischichtig ausgebildet ist.

Fig. 7 zeigt in entsprechender perspektivischer Ansicht nur das Innenteil 100 aus Cycloolefin.

Die Stirnwand 103 des Innenteils 100 umfasst eine Strukturierung 104, welche vorzugsweise durch den Spritzguss bereitgestellt wurde.

In diesem Ausführungsbeispiel umfasst die Strukturierung 104 der Stirnwand 103 eine Vielzahl von sich konzentrisch um die Düse 101 des Innenteils 100 erstreckende Ringe 105a - 105n, welche als Berge bzw. Erhebungen ausgebildet sind.

Diese Ringe 105a - 105n werden durch eine Vielzahl von sich radial erstreckenden Nuten 106 unterbrochen, welche im Zusammenwirken mit dem Außenteil 200 eine Verdrehsicherung bilden.

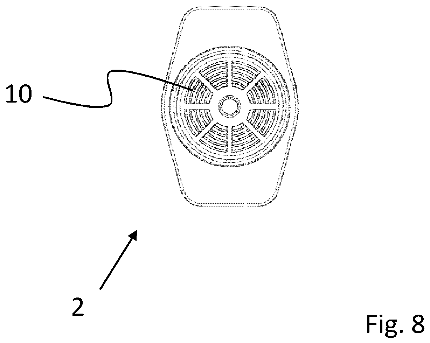

Fig. 8 ist eine Draufsicht auf die distale Seite der Spritzenkörpers 2.

Die Stirnwand des Spitzenkörpers 2 umfasst einen zweischichtig ausgebildeten Bereich 7, in welchem die Strukturierungen 104, 208 von Innenteil 100 und Außenteil 200 ineinandergreifen.

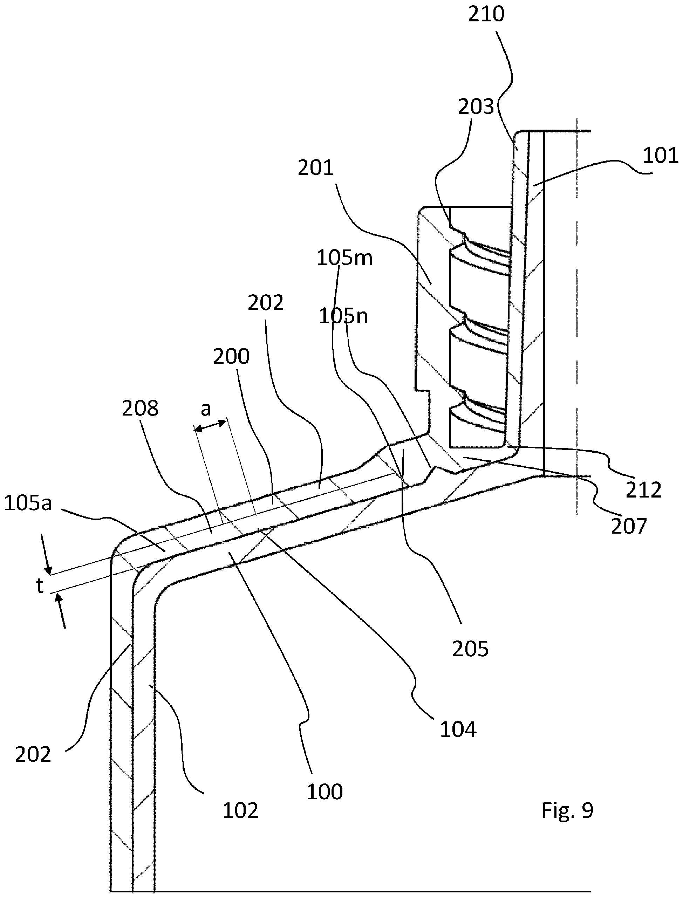

Die erfindungsgemäß ausgebildete ineinandergreifende Strukturierung wird also, wie insbesondere in der Detaildarstellung gemäß Fig. 9 entlang der Schnittlinie B gemäß Fig. 5 gezeigt, durch die Strukturierung 104 des Innenteils 100 sowie durch die in diese Strukturierung 104 eingreifende Strukturierung 208 der Stirnwand 211 des Außenteils 200 gebildet.

Die Strukturierung 104 des Innenteils 100 umfasst in einer radialen Schnittansicht eine Vielzahl von Zähnen 105a - 105n. Die Strukturierung 104 ist also in diesem Ausführungsbeispiel in einem axialen Schnitt als Sägezahnprofil ausgebildet.

Die korrespondierend ausgebildeten Zähne des zweiten Materials des Außenteils 200 greifen zwischen die Zähne 105a - 105n ein.

Das zweite Material erstreckt sich um die Außenwände des gesamten Innenteils 100.

Die Strukturierung (104/208) ist in diesem Ausführungsbeispiel als regelmäßige Strukturierung ausgebildet, bei welcher der Abstand Berg zu Berg bzw. Tal zu Tal (bzw. Erhebung zu Erhebung oder Vertiefung zu Vertiefung), also die Strukturbreite, durch den Abstand a zwischen zwei Spitzen oder zwei Tälern definiert ist.

Weiter hat die Strukturierung 104/208 eine Maximaltiefe t, durch die die Strukturtiefe definiert wird, und die durch den vertikalen Abstand Spitze Berg und Grund Tal definiert ist.

Der Gewindeanschluss 201 mit den Zähnen 203 des Innengewindes, welches der Düse 210 gegenüberliegt, geht über einen Wulst 205 im Bereich des zweiten Materials in die Stirnwand 202 des Außenteils 200 über.

Hierdurch wird die mechanische Festigkeit in diesem Bereich erhöht, so dass das Risiko eines Abbrechens des Gewindeanschlusses 201 reduziert wird.

Innerhalb des Gewindeanschlusses 201 reicht der Grund 207 des Gewindeanschlusses 201 bis zu einer inneren Ecke 212, an welcher die Stirnwand 211 des Außenteils 200 in die Düse 210 übergeht.

Die Düse 4 ist also zweischichtig ausgebildet, und zwar aus der Düse 101 des Innenteils 100, welche die Innenwand der Düse 4 bereitstellt und aus der Düse 210 des Außenteils 200, welche die Außenwand der Düse 4 bereitstellt.

Fig. 7 ist eine Schnittansicht entlang der Linie C der Fig. 5.

Nunmehr verläuft der Schnitt durch eine radiale Nut 106 des Innenteils 100. Die Nut 106 wird dadurch gebildet, dass in einem sich radial erstreckenden Streifen die Zähne bzw. Ringe 105a -105m der Strukturierung 104 unterbrochen sind.

In der Nut 106 ist ein korrespondierender Steg 209 aus dem zweiten Material ausgebildet.

Dieser bildet im Zusammenwirken mit der Nut 106 eine Verdrehsicherung.

In diesem Ausführungsbeispiel ist ein direkt an den Gewindeanschluss 201 reichender Ring 105n nicht durch die Nut 106 ausgespart.

Dieser bildet auch im Bereich des Stegs 209 eine Umlenkstelle, über die der Eintrag von Spannungen in den Bereich des Stegs 209 reduziert wird.

Innenteil 100 und Außenteil 200 können stoffschlüssig verbunden sein, insbesondere indem das Innenteil 100 mit dem Außenteil 200 umspritzt ist. Alternativ ist möglich, Innenteil 100 und Außenteil 200 durch Schweißen oder Kleben oder durch eine form- und/oder kraftschlüssige Verbindung miteinander zu verbinden.

Durch die Erfindung könnte eine Spritze 1 bereitgestellt werden, welche stabiler ist, insbesondere weniger zu Sprödbruch neigt und eine verbesserte Haptik hat. Gleichzeitig konnte die notwendige Menge an Cycloolefin verringert werden.

### Bezugszeichenliste

- 1: Spritze
- 2: Spritzenkörper
- 3: Innenvolumen
- 4: Düse
- 5: Gewindeanschluss
- 6: Kolben
- 7: Griff
- 8: Kolbenstange
- 9: Dichtung
- 10: zweischichtiger Bereich mit Strukturierung
- 100: Innenteil
- 101: Düse
- 102: Seitenwand
- 103: Stirnwand
- 104: Strukturierung des Innenteils
- 105a-105n: Ring/Zahn
- 106: radiale Nut
- 107: Kragen
- 200: Außenteil
- 201: Gewindeanschluss
- 202: Seitenwand
- 203: Zahn
- 204: Griff
- 205: Wulst
- 206: Oberseite
- 207: Grund
- 208: Strukturierung des Außenteils
- 209: Steg
- 210: Düse
- 211: Stirnwand
- 212: Ecke

## Patentansprüche

1. Spritzenkörper (2) für eine medizinische Spritze (1) umfassend eine Düse (4) und einen Gewindeanschluss (5), wobei der Spritzenkörper (2) aus einem Innenteil (100) und einem Außenteil (200) gebildet wird,
wobei das Innenteil (100) eine Innenwand des Spritzenkörpers (2) ausbildet und ein Cycloolefin als erstes Material umfasst,
wobei der Gewindeanschluss (5) als Teil des Außenteils (200) aus einem Kunststoff ausgebildet ist, welcher als zweites Material ein anderes Material als das Cycloolefin umfasst, wobei
die Düse (4) zweischichtig ausgebildet ist, aus der Düse (101) des Innenteils (100), welche die Innenwand der Düse (4) bereitstellt, und aus der Düse (210) des Außenteils (200), welche die Außenwand der Düse (4) bereitstellt, und die Seitenwand des Spritzenkörpers (2) zweischichtig ausgebildet ist und aus einer Seitenwand (102) des Innenteils (100) und einer angrenzenden Seitenwand (202) des Außenteils (200) gebildet wird, wobei sich das Außenteil (200) zumindest abschnittsweise um die Seitenwand (102) des Innenteils (100) erstreckt.

2. Spritzenkörper (2) nach dem vorstehenden Anspruch, **dadurch gekennzeichnet, dass** sich das Außenteil (200) um die gesamte Seitenwand (102) des Innenteils (100) erstreckt und/oder dass ein Griff des Spritzenkörpers (2) Teil des Außenteils (200) ist.

3. Spritzenkörper (2) nach einem der vorstehenden Ansprüche, **dadurch gekennzeichnet, dass** das Innenteil (100) und das Außenteil (200) ineinandergesteckt sind und insbesondere zumindest abschnittsweise stoffschlüssig verbunden sind.

4. Spritzenkörper (2) nach einem der vorstehenden Ansprüche, **dadurch gekennzeichnet, dass** das Innenteil (100) mit dem Außenteil (200) umspritzt ist.

5. Spritzenkörper (2) nach einem der vorstehenden Ansprüche, **dadurch gekennzeichnet, dass** die Wandstärke des Innenteils (100), insbesondere im Bereich der Seitenwand und/oder der Stirnwand, das 0,3 bis 3fache, vorzugsweise das 0,5 bis 1,5fache, der Wandstärke der angrenzenden Wand des Außenteils (200) beträgt.

6. Spritzenkörper (2) nach einem der vorstehenden Ansprüche, **dadurch gekennzeichnet, dass** eine Vorderseite einer Stirnwand (103) des Innenteils (100) eine Strukturierung (104) umfasst, in die eine komplementäre Strukturierung (208) des Außenteils greift.

7. Spritzenkörper (2) nach einem der vorstehenden Ansprüche, **dadurch gekennzeichnet, dass** die Strukturierung (104/208) des Innenteil (100) und/oder des Außenteils (200) jeweils als ein Profil mit einer Vielzahl von Erhebungen und/oder Vertiefungen ausgebildet ist.

8. Spritzenkörper (2) nach einem der vorstehenden Ansprüche, **dadurch gekennzeichnet, dass** die Strukturierung zumindest eine sich radial erstreckende Nut oder einen sich radial erstreckenden Steg, vorzugsweise eine Vielzahl von sich radial erstreckenden Stegen (209) und/oder Nuten (106), aufweist.

9. Spritzenkörper (2) nach einem der vorstehenden Ansprüche, **dadurch gekennzeichnet, dass** eine Schicht aus dem zweiten Material um den Gewindeanschluss (5) herum verdickt gegenüber einem radial angrenzenden Bereich der Schicht aus dem zweiten Material ausgebildet ist.

10. Spritzenkörper (2) nach einem der vorstehenden Ansprüche, **dadurch gekennzeichnet, dass** das zweite Material eine höhere Kerbschlagzähigkeit als das erste Material aufweist, insbesondere eine mindestens 20 %, vorzugsweise mindestens 50 %, höhere Kerbschlagzähigkeit als das erste Material aufweist.

11. Spritzenkörper (2) nach einem der vorstehenden Ansprüche, **dadurch gekennzeichnet, dass** das zweite Material weicher als das erste Material ist.

12. Spritzenkörper (2) nach einem der vorstehenden Ansprüche, **dadurch gekennzeichnet, dass** die Strukturierung an der Vorderseite der Stirnwand (103) des Innenteils (100) durch einen radial umlaufenden Steg begrenzt ist.

13. Spritze (1), umfassend einen Spritzenkörper (2) nach einem der vorstehenden Ansprüche.

14. Spritze (1) nach einem der vorstehenden Ansprüche, **dadurch gekennzeichnet, dass** die Düse (5) mit einer, vorzugsweise in die Düse (5) greifenden, Dichtung (9) verschlossen ist, und dass die Spritzen einen Kolben (6) mit einer Kolbenstange (8) zur Abgabe einer medizinischen Flüssigkeit durch die Düse (4) umfasst.

15. Spritze (1) nach einem der vorstehenden Ansprüche, **dadurch gekennzeichnet, dass** die Spritze in einer für Sauerstoff impermeablen Überverpackung angeordnet ist.

## Claims

1. A syringe body (2) for a medical syringe (1), comprising a nozzle (4) and a threaded connection (5), wherein the syringe body (2) is formed from an inner part (100) and an outer part (200),
wherein the inner part (100) forms an inner wall of the syringe body (2) and comprises a cycloolefin as the first material,
wherein the threaded connection (5) is formed as part of the outer part (200) from a plastic which comprises a material other than the cycloolefin as the second material,
wherein the nozzle (4) is formed in two layers, from the nozzle (101) of the inner part (100), which provides the inner wall of the nozzle (4), and from the nozzle (210) of the outer part (200) which provides the outer wall of the nozzle (4) and the side wall of the syringe body (2) is formed in two layers and is formed from a side wall (102) of the inner part (100) and an adjoining side wall (202) of the outer part (200), wherein the outer part (200) extends at least in sections around the side wall (102) of the inner part (100).

2. The syringe body (2) according to the preceding claim, **characterised in that** the outer part (200) extends around the entire side wall (102) of the inner part (100) and/or **in that** a handle of the syringe body (2) is part of the outer part (200).

3. The syringe body (2) according to one of the preceding claims, **characterised in that** the inner part (100) and the outer part (200) are inserted into one another and, in particular, are connected at least in sections in a materially-bonded manner.

4. The syringe body (2) according to one of the preceding claims, **characterised in that** the inner part (100) is overmoulded with the outer part (200).

5. The syringe body (2) according to one of the preceding claims, **characterised in that** the wall thickness of the inner part (100), in particular in the region of the side wall and/or the end wall, is 0.3 to 3 times, preferably 0.5 to 1.5 times, the wall thickness of the adjoining wall of the outer part (200).

6. The syringe body (2) according to one of the preceding claims, **characterised in that** a front side of an end wall (103) of the inner part (100) comprises a structuring (104) into which a complementary structuring (208) of the outer part engages.

7. The syringe body (2) according to one of the preceding claims, **characterised in that** the structuring (104/208) of the inner part (100) and/or of the outer part (200) is each in the form of a profile with a plurality of elevations and/or depressions.

8. The syringe body (2) according to one of the preceding claims, **characterised in that** the structuring has at least one radially extending groove or a radially extending web, preferably a plurality of radially extending webs (209) and/or grooves (106).

9. The syringe body (2) according to one of the preceding claims, **characterised in that** a layer of the second material around the threaded connection (5) is thickened relative to a radially adjoining region of the layer of the second material.

10. The syringe body (2) according to one of the preceding claims, **characterised in that** the second material has a higher notched impact strength than the first material, in particular at least 20%, preferably at least 50%, higher notched impact strength than the first material.

11. The syringe body (2) according to one of the preceding claims, **characterised in that** the second material is softer than the first material.

12. The syringe body (2) according to one of the preceding claims, **characterised in that** the structuring on the front side of the end wall (103) of the inner part (100) is delimited by a radially circumferential web.

13. A syringe (1) comprising a syringe body (2) according to one of the preceding claims.

14. The syringe (1) according to one of the preceding claims, **characterised in that** the nozzle (5) is closed with a seal (9), preferably engaging in the nozzle (5), and **in that** the syringe comprises a piston (6) with a piston rod (8) for dispensing a medical liquid through the nozzle (4).

15. The syringe (1) according to one of the preceding claims, **characterised in that** the syringe is arranged in an oxygen-impermeable outer package.

## Revendications

1. Corps de seringue (2) pour une seringue médicale (1), comprenant une buse (4) et un raccord fileté (5), dans lequel le corps de seringue (2) est formé à partir d'une partie interne (100) et d'une partie externe (200),
dans lequel la partie interne (100) crée une paroi interne du corps de seringue (2) et comprend une cyclooléfine en guise de premier matériau,
dans lequel le raccord fileté (5) est créé en guise de partie de la partie externe (200) à partir d'une matière plastique, qui comprend en guise de second matériau un autre matériau différent que la cyclooléfine,
dans lequel la buse (4) est créée en deux couches, à partir de la buse (101) de la partie interne (100), qui fournit la paroi interne de la buse (4), et à partir de la buse (210) de la partie externe (200), qui fournit la paroi externe de la buse (4), et la paroi latérale du corps de seringue (2) est créée en deux couches et est formée à partir d'une paroi latérale (102) de la partie interne (100) et d'une paroi latérale adjacente (202) de la partie externe (200), dans lequel la partie externe (200) s'étend au moins par sections autour de la paroi latérale (102) de la partie interne (100).

2. Corps de seringue (2) selon la revendication précédente, **caractérisé en ce que** la partie externe (200) s'étend autour de toute la paroi latérale (102) de la partie interne (100) et/ou **en ce qu'**une poignée du corps de seringue (2) fait partie de la partie externe (200).

3. Corps de seringue (2) selon l'une des revendications précédentes, **caractérisé en ce que** la partie interne (100) et la partie externe (200) sont emboîtées l'une dans l'autre et sont en particulier reliées au moins par sections par une liaison de matière.

4. Corps de seringue (2) selon l'une des revendications précédentes, **caractérisé en ce que** la partie interne (100) est surmoulée avec la partie externe (200).

5. Corps de seringue (2) selon l'une des revendications précédentes, **caractérisé en ce que** l'épaisseur de paroi de la partie interne (100), en particulier dans la zone de la paroi latérale et/ou de la paroi frontale, est de 0,3 à 3 fois, de préférence de 0,5 à 1,5 fois, l'épaisseur de paroi de la paroi adjacente de la partie externe (200).

6. Corps de seringue (2) selon l'une des revendications précédentes, **caractérisé en ce qu'**un côté avant d'une paroi frontale (103) de la partie interne (100) comprend une structuration (104) avec laquelle vient en prise une structuration complémentaire (208) de la partie externe.

7. Corps de seringue (2) selon l'une des revendications précédentes, **caractérisé en ce que** la structuration (104/208) de la partie interne (100) et/ou de la partie externe (200) est créée respectivement sous la forme d'un profil comportant une pluralité de bosses et/ou de creux.

8. Corps de seringue (2) selon l'une des revendications précédentes, **caractérisé en ce que** la structuration présente au moins une rainure s'étendant radialement ou une nervure s'étendant radialement, de préférence une pluralité de nervures (209) et/ou rainures (106) s'étendant radialement.

9. Corps de seringue (2) selon l'une des revendications précédentes, **caractérisé en ce qu'**une couche à partir du second matériau est créée autour du raccord fileté (5) de manière épaissie par rapport à une zone radialement adjacente de la couche à partir du second matériau.

10. Corps de seringue (2) selon l'une des revendications précédentes, **caractérisé en ce que** le second matériau présente une résilience plus élevée que le premier matériau, en particulier une résilience supérieure d'au moins 20 %, de préférence d'au moins 50 %, à celle du premier matériau.

11. Corps de seringue (2) selon l'une des revendications précédentes, **caractérisé en ce que** le second matériau est plus mou que le premier matériau.

12. Corps de seringue (2) selon l'une des revendications précédentes, **caractérisé en ce que** la structuration est limitée sur le côté avant de la paroi frontale (103) de la partie interne (100) par une nervure périphérique radiale.

13. Seringue (1), comprenant un corps de seringue (2) selon l'une des revendications précédentes.

14. Seringue (1) selon l'une des revendications précédentes, **caractérisée en ce que** la buse (5) est fermée par un joint d'étanchéité (9) venant en prise de préférence dans la buse (5), et **en ce que** la seringue comprend un piston (6) comportant une tige de piston (8) pour délivrer un liquide médical à travers la buse (4).

15. Seringue (1) selon l'une des revendications précédentes, **caractérisée en ce que** la seringue est agencée dans un suremballage imperméable à l'oxygène.
